# EUROPEAN PATENT SPECIFICATION

(11) **EP 3 766 484 B1**
(45) Date of publication and mention of the grant of the patent: **25.08.2021**
(21) Application number: 19187219.1
(22) Date of filing: 19.07.2019
(51) Int. Cl.: A61K 9/16, A61K 9/20, A61K 9/24, A61K 31/216, A61K 31/41

(54) **SOLID PHARMACEUTICAL DOSAGE FORM COMPRISING VALSARTAN AND SACUBITRIL**
FESTE PHARMAZEUTISCHE DARREICHUNGSFORM MIT VALSARTAN UND SAKUBITRIL
FORME POSOLOGIQUE PHARMACEUTIQUE SOLIDE COMPRENANT DU VALSARTAN ET DU SACUBITRIL

(43) Date of publication of application: 20.01.2021
(73) Proprietor: ZENTIVA, K.S., 10 237 Praha 10 (CZ)
(72) Inventor: SEDMAK, Gregor, 10200 Prague 10 (CZ); BERANEK, Josef, 16000 Prague 6 (CZ)
(74) Representative: Hartvichova, Katerina

(56) References cited:
- WO-A1-2017/012600
- WO-A1-2019/008485

## Description

### Technical Field

The present invention relates to a solid pharmaceutical dosage form comprising (2S)-3-methyl-2-[pentanoyl[[2'-(1H-tetrazol-5-yl)biphenyl-4-yl]methyl]amino]butanoic acid, also known as valsartan, its chemical formula being 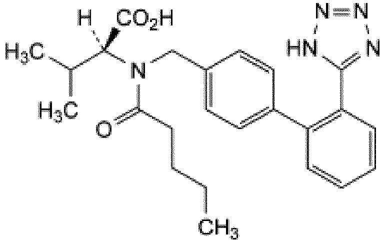 in a form of valsartan disodium salt hydrate or in a form of free acid; and 4-{[(2S,4R)-1-([1,1'-biphenyl]-4-yl)-5-ethoxy-4-methyl-5-oxopentan-2-yl]amino}-4-oxobutanoic acid, also known as sacubitril, its chemical formula being 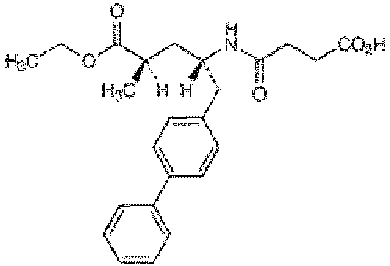 in a form of sacubitril sodium salt and a process of preparation of the pharmaceutical dosage form according to the invention.

### Background of the Invention

Valsartan is a substance with a very poor water solubility at pH below 5. Above pH 5, both sacubitril and valsartan exhibit high solubility in aqueous medium. Valsartan as a mono component has been on the market for a long time under the commercial name Diovan. In this product, valsartan is present in a free acid form as disclosed in Diovan SPC. On the other hand, in case of a combination product with sacubitril, valsartan is present in a form of a sodium salt complex with sacubitril as disclosed in Entresto : EPAR - Procedural steps taken and scientific information after authorization, WO2007056546 and WO2009061713. WO2007056546 and WO2009061713 disclose that though combination product of valsartan and sacubitril in the form of valsartan-sacubitril complex may be prepared by a simple direct compression technique, it is preferably prepared by a more complicated roller compaction process.

The drawback of a formulation in which valsartan and sacubitril are present in the form of a complex is that both active ingredients behave like a single unity and thus dissolve as a single component at the same time. However, in order to have a sufficient flexibility in dissolution profile of each component, it is beneficial to have valsartan and sacubitril separated as two distinct components. In that way, the dissolution profile of each component can be tailored as needed.

WO2017012600 discloses products containing valsartan and sacubitril in the form of free acid or ester, respectively, or in the form of salts, as separate molecules. Lack of stability of sacubitril was noted therein. All compositions were produced by mixing the active ingredient or mixture of active ingredients with all excipients at once. When each API was mixed with excipients separately, then wet granulation was used for valsartan.

WO2019/008485 discloses pharmaceutical compositions comprising fixed dose combination of valsartan or pharmaceutically acceptable salts thereof and sacubitril or pharmaceutically acceptable salts thereof.

### Disclosure of the Invention

Within the framework of the invention, it has been surprisingly found that it is possible to prepare a stable, robust and fast dissolving tablet composition comprising valsartan disodium salt hydrate or valsartan free acid, and sacubitril sodium salt, respectively, in separate particles by employing a dry manufacturing process. Additionally, the mixture prepared as described in the invention has significantly improved flow properties for tableting.

The dry manufacturing process is preferably selected from roller compaction and slugging. It is well known in the art of pharmaceutical process development that standard mode of roller compaction or slugging is performed in such a way that the active ingredient is roller compacted or slugged together with filler, binder, disintegrant and optionally glidant and lubricant. For example, Remington (J.P. Remington, A. R. Gennaro, Remington : the science and practice of pharmacy, 20th ed. Baltimore, Md. : Lippincott Williams & Wilkins, ©2000) teaches that dry granulation is performed by unit operations of weighing, mixing, compaction, dry screening, lubrication and tablet compression. It is also well known in the art of pharmaceutical process development that the addition of a lubricant into the roller compaction or slugging process may adversely affect dissolution characteristics of the resulting tablets and is therefore avoided if possible. However, if a lubricant is needed inside the compactate, it is commonly used in as low amount as possible, typically in the amounts of 0.5 w/w% or less.

Within the framework of the present invention, it has surprisingly been found that a special mode of roller compaction or slugging gives optimal results. In this mode, each of valsartan (in the form of disodium salt hydrate or in the form of free acid) and sacubitril sodium salt is roller compacted or slugged with only addition of at least one lubricant and optionally with at least one glidant (flow agent), without fillers, binders or disintegrants. Fillers, and binders and disintegrants (if present), are contained in the extra-particular phase, i.e., in the phase outside the particles.

In one aspect of the present invention, a composition is provided, comprising
- particles containing valsartan (in the form of disodium salt hydrate or in the form of free acid), at least one lubricant and optionally at least one glidant, but not containing fillers, binders, nor disintegrants;
- particles containing sacubitril sodium salt, at least one lubricant and optionally at least one glidant, but not containing fillers, binders, nor disintegrants;
- extra-particular phase containing at least one filler, and optionally at least one binder and/or optionally at least one disintegrant.

Preferably, the particles are granules, slugs or compactates. In a preferred embodiment, the particles may have the size within the range of 0.01 to 5 mm, more preferably up to 2 mm, or 0.4 to 1.5 mm (wherein at least 80 % of the particles are within the relevant size range).

In another aspect of the present invention, the composition comprising valsartan (in the form of disodium salt hydrate or in the form of free acid) and sacubitril sodium salt, respectively, in separate particles is formulated into the form of a single layer tablet.

When the composition comprising valsartan (in the form of disodium salt hydrate or in the form of free acid) and sacubitril sodium salt, respectively, as separate particles is prepared by roller compaction or slugging process and formulated into the form of a single layer tablet, the process contains the following steps:
1. mixing of valsartan (in the form of disodium salt hydrate or in the form of free acid) with at least one lubricant and optionally with at least one glidant;
2. roller compaction or slugging of the mixture containing valsartan (in the form of disodium salt hydrate or in the form of free acid) into compactate or slugs;
3. sieving or milling the compactate or slugs into particles (e.g., granulate) containing valsartan (in the form of disodium salt hydrate or in the form of free acid);
4. mixing of sacubitril sodium salt with at least one lubricant and optionally with at least one glidant;
5. roller compaction or slugging of the mixture containing sacubitril sodium salt into compactate or slugs;
6. sieving or milling the compactate or slugs into particles (e.g. granulate) containing sacubitril sodium salt;
7. mixing of the particles containing valsartan (in the form of disodium salt hydrate or in the form of free acid) and the particles containing sacubitril sodium salt;
8. admixing further excipients (e.g., fillers, binders, disintegrants) to prepare a mixture ready for tableting;
9. compressing the said mixture into a tablet.

In another aspect of the present invention, the composition comprising valsartan (in the form of disodium salt hydrate or in the form of free acid) and sacubitril sodium salt, respectively, in separate particles are formulated into the form of a multilayer tablet. The multilayer tablet has at least two layers, a layer comprising valsartan (in the form of disodium salt hydrate or in the form of free acid)-containing particles and a layer comprising sacubitril sodium salt-containing particles.

In a preferred embodiment, the multilayer tablet comprising valsartan (in the form of disodium salt hydrate or in the form of free acid) and sacubitril sodium salt in separate particles and in separate layers may optionally include an additional separation layer.

When the composition comprising valsartan (in the form of disodium salt hydrate or in the form of free acid) and sacubitril sodium salt, respectively, as separate particles is prepared by roller compaction or slugging process and formulated into the form of a multilayer tablet, the process contains the following steps:
1. mixing of valsartan (in the form of disodium salt hydrate or in the form of free acid) with at least one lubricant and optionally with at least one glidant;
2. roller compaction or slugging of the mixture containing valsartan (in the form of disodium salt hydrate or in the form of free acid) into compactate or slugs;
3. sieving or milling the compactate or slugs into particles (e.g. granulate) containing valsartan (in the form of disodium salt hydrate or in the form of free acid);
4. admixing optional further excipients (such as fillers, binders, disintegrants) in order to prepare a mixture containing valsartan (in the form of disodium salt hydrate or in the form of free acid) ready for tableting;
5. mixing of sacubitril sodium salt with at least one lubricant and optionally with at least one glidant;
6. roller compaction or slugging of the mixture containing sacubitril sodium salt into compactate or slugs;
7. sieving or milling the compactate or slugs into particles (e.g. granulate) containing sacubitril sodium salt;
8. admixing optional further excipients (such as fillers, binders, disintegrants) in order to prepare a mixture containing sacubitril sodium salt ready for tableting;
9. optionally preparing a separation-layer mixture not containing any active ingredient;
10. compressing the said valsartan (in the form of disodium salt hydrate or in the form of free acid) mixture, the sacubitril sodium salt mixture and the optional separation mixture not containing any active ingredient into a multilayer tablet.

In one aspect of the present invention, the particles (e.g. granulate) containing valsartan (in the form of disodium salt hydrate or in the form of free acid) typically contain the at least one lubricant in the total amount of 0.5 w/w% or more. A particularly preferred amount of the lubricant(s) in the particles containing valsartan (in the form of disodium salt hydrate or in the form of free acid) is 1 w/w% or more.

In one aspect of the present invention, the particles (e.g. granulate) containing sacubitril sodium salt typically contain the at least one lubricant in the total amount of 0.5 w/w% or more. A particularly preferred amount of the lubricant(s) in the particles containing sacubitril sodium salt is 1 w/w% or more.

In one aspect of the present invention, the composition comprising valsartan (in the form of disodium salt hydrate or in the form of free acid) and sacubitril sodium salt, respectively, as separate particles typically contains a further extraparticular (e.g. extragranular) amount of at least one lubricant in the total amount of 0.5 w/w% or more. A particularly preferred further extraparticular amount of the lubricant(s) in the composition comprising valsartan (in the form of disodium salt hydrate or in the form of free acid) and sacubitril sodium salt, respectively, in separate particles is 1% w/w% or more.

The composition comprising valsartan (in the form of disodium salt hydrate or in the form of free acid) and sacubitril sodium salt, respectively, as separate particles typically comprises optional further excipients in the extraparticular phase. Suitable further optional excipients may be selected from fillers, binders, disintegrants, lubricants, glidants and coating agents, in particular at least one filler and at least one disintegrant.

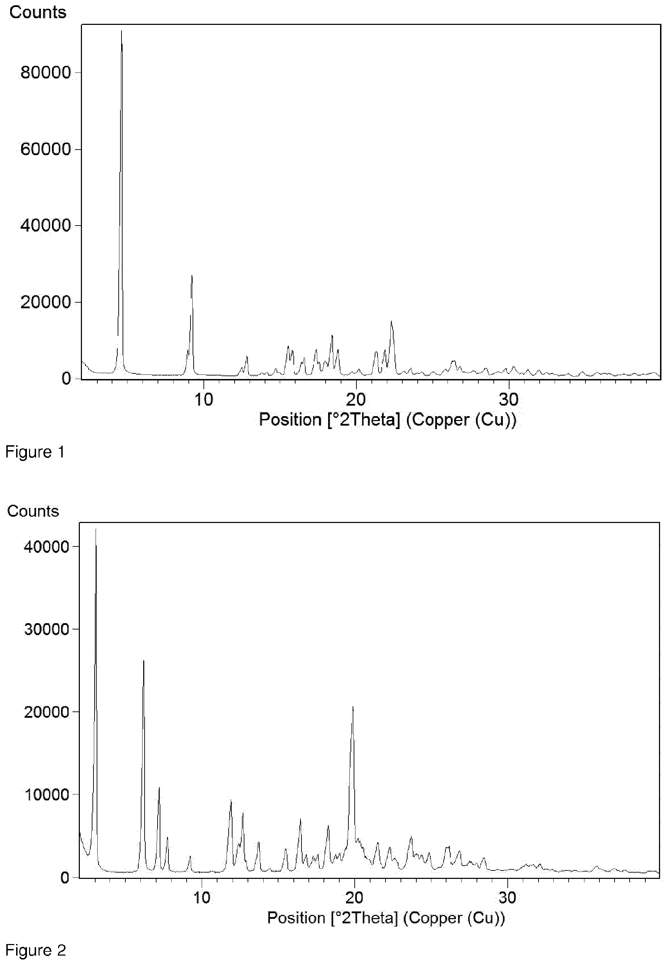

In one aspect of the invention, the composition comprises crystalline valsartan disodium salt hydrate, defined as Form I, characterized by an X-ray powder diffraction pattern having peaks at about 4.6; 9.3; 12.8; 15.5; 18.4 and 22.3 degrees two theta ± 0.2 degrees two theta. X-ray powder diffraction pattern is depicted in Figure 1. Form I of valsartan disodium salt hydrate may be further characterized by X-ray powder diffraction pattern having additional peaks at about 16.6; 17.4; 18.8; 21.3; 21.9 and 26.4 degrees two theta ± 0.2 degrees two theta. This crystalline form and its preparation was described in WO 02/06253, in particular example 11.

Form I of crystalline valsartan disodium salt hydrate is non-stoichiometric hydrate and may contain 9 to 15 % of water, preferably between 10 and 13 % and most preferably between 10 and 11.5 % (% by weight).

In one aspect of the invention, the composition comprises crystalline sacubitril sodium salt, defined as Form I, characterized by an X-ray powder diffraction pattern having peaks at about 3.1; 6.2; 11.9; 16.4; 18.3 and 19.9 degrees two theta ± 0.2 degrees two theta. X-ray powder diffraction pattern is depicted in Figure 2. Form I of sacubitril sodium salt may be further characterized by X-ray powder diffraction pattern having additional peaks at about 7.2; 12.7; 15.5; 21.5; 22.3 and 23.7 degrees two theta ± 0.2 degrees two theta. This crystalline form and its preparation was described in CZ PV 2015-896.

A suitable filler useful for preparation of valsartan (in the form of disodium salt hydrate or in the form of free acid) and sacubitril sodium salt tablets may be selected from water soluble fillers, water insoluble fillers and mixtures thereof. Water soluble fillers may include hydroxypropyl cellulose, oligosaccharides, monosaccharides and sugar alcohols, more preferably mannitol, trehalose, erythritol, fructose, glucose, saccharose, raffinose, isomalt, dextrates, lactose anhydrous, lactose monohydrate, sorbitol, maltitol, lactose anhydrous, xylitol and lactitol. Water insoluble fillers may include microcrystalline cellulose, silicified microcrystalline cellulose, calcium hydrogen phosphate anhydrous, calcium hydrogen phosphate dihydrate.

In another aspect of the present invention, the tablets comprising valsartan (in the form of disodium salt hydrate or in the form of free acid) and sacubitril sodium salt in separate particles exhibit a beneficial stability profile if a filler with reduced moisture content is used. Fillers with reduced moisture content typically include mannitol, lactose anhydrous, microcrystalline cellulose, silicified microcrystalline cellulose and calcium hydrogen phosphate anhydrous. In a particularly preferred embodiment, microcrystalline cellulose and silicified microcrystalline cellulose are used.

A suitable binder (if present) useful for preparation of valsartan (in the form of disodium salt hydrate or in the form of free acid) and sacubitril sodium salt tablets may be selected from pregelatinized starch, polymethacrylates, copovidone, povidone, maltose, starch, cellulose powder, crystalline cellulose, microcrystalline cellulose, silicified microcrystalline cellulose, cellulose derivatives such as hydroxymethylcellulose, hydroxyethylcellulose, hydroxypropyl cellulose, low-substituted hydroxypropyl cellulose and hydroxypropylmethylcellulose, and mixtures thereof. Preferred are microcrystalline cellulose and low-substituted hydroxypropyl cellulose.

A suitable disintegrant (if present) useful for preparation of valsartan (in the form of disodium salt hydrate or in the form of free acid) and sacubitril sodium salt tablets may be selected from croscarmellose sodium, crospovidone, hydroxypropyl starch, low-substituted hydroxypropyl cellulose, sodium starch glycolate and mixtures thereof. Crospovidone and low-substituted hydroxypropyl cellulose are particularly preferred.

A suitable lubricant used for preparation of valsartan (in the form of disodium salt hydrate or in the form of free acid) particles and sacubitril sodium salt particles may be selected from hydrophobic lubricants such as magnesium stearate, talc, calcium stearate, stearic acid and hydrogenated vegetable oil, and hydrophilic lubricants such as glyceryl behenate, sodium stearyl fumarate, polyethylene glycol, sodium lauryl sulphate, isoleucine and sodium benzoate. Particularly preferred is magnesium stearate and/or talc.

A suitable glidant used for preparation of valsartan (in the form of disodium salt hydrate or in the form of free acid) particles and sacubitril sodium salt particles may be selected from colloidal silicon dioxide, hydrophobic colloidal silica (anhydrous colloidal silica), magnesium oxide, magnesium silicate, magnesium trisilicate, talc, calcium silicate and tribasic calcium phosphate. Particularly preferred are anhydrous colloidal silica and talc.

The tablets may be coated. A suitable coating agent useful for preparation of valsartan (in the form of disodium salt hydrate or in the form of free acid) and sacubitril sodium salt tablets may be selected from hypromellose, hydroxypropylcellulose, macrogol, polyvinyl alcohol, polyethylene glycol and mixtures thereof. Particularly preferred is hypromellose. The coating may further include lubricants (e.g., talc) and pigments (e.g. titanium dioxide, iron oxide).

### Figures

Figure 1 depicts the powder X-ray diffraction spectrum of crystalline Valsartan disodium salt hydrate, defined as Form I.
Figure 2 depicts the powder X-ray diffraction spectrum of crystalline Sacubitril sodium salt, defined as Form I.

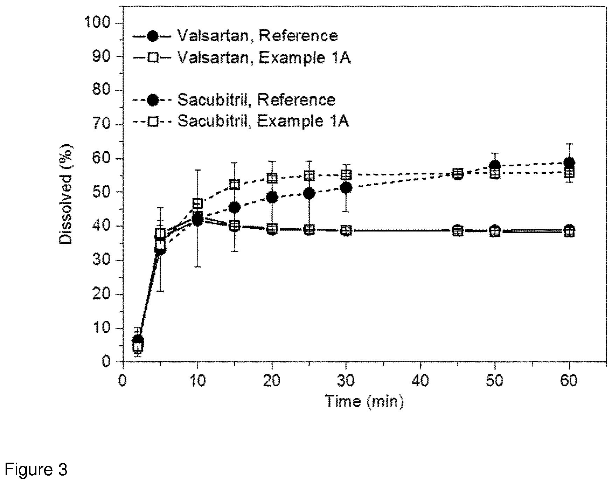

Figure 3 depicts dissolution of a prototype according to Example 1A in comparison to the reference product according to Comparative Example 1.

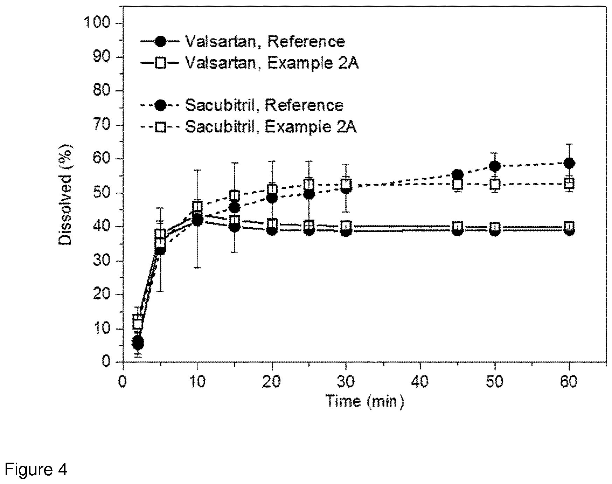

Figure 4 depicts dissolution of a prototype according to Example 2A in comparison to the reference product according to Comparative Example 2.

### Examples of carrying out the Invention

The following section contains examples relative to the present invention, as well as comparative examples, in order to better illustrate the advantages of the present invention.

### Comparative example 1. Composition comprising valsartan disodium salt hydrate and sacubitril sodium salt prepared in the form of a single layer tablet

Valsartan disodium salt hydrate, sacubitril sodium salt, filler, binder and a disintegrant are roller compacted and sieved thru 1 mm sieve. To the said sieved granulate, glidant and lubricant are admixed in order to prepare a mixture ready for tableting. The said tableting mixture is compressed into tablets on a high speed tableting machine with round, lentil shaped tableting punches of 11 mm diameter.

| | | A | B | C | D |
|---|---|---|---|---|---|
| Valsartan disodium salt hydrate | active | 124.50 | 124.50 | 124.50 | 124.50 |
| Sacubitril sodium salt | active | 102.40 | 102.40 | 102.40 | 102.40 |
| Microcrystalline cellulose | filler | 77.10 | | 87.10 | |
| Calcium hydrogen phosphate anhydrous | filler | | 77.10 | | 87.10 |
| Low-substituted hydroxypropyl cellulose | binder | 60.00 | 60.00 | 30.00 | 30.00 |
| Crospovidone | disintegrant | 20.00 | 20.00 | 40.00 | 40.00 |
| Colloidal silicon dioxide | glidant | 4.00 | 4.00 | 4.00 | 4.00 |
| Talc | glidant | 8.00 | 8.00 | 8.00 | 8.00 |
| Magnesium stearate | lubricant | 4.00 | 4.00 | 4.00 | 4.00 |
| Total single layer tablet (mg) | | 400.00 | 400.00 | 400.00 | 400.00 |

During the tableting process, tableting mixture exhibits a poor material flow into the tableting die, resulting in a high tablet mass variability, which corresponds to low content uniformity of both active ingredients.

### Example 1A. Composition comprising valsartan disodium salt hydrate and sacubitril sodium salt as separate units prepared in the form of a single layer tablet

Valsartan disodium salt hydrate, glidants and lubricant are mixed, roller compacted and sieved thru 1 mm sieve in order to obtain granulate containing valsartan disodium salt hydrate of desired particle size. Sacubitril sodium salt, glidants and lubricant are mixed, roller compacted and sieved thru 1 mm sieve in order to obtain granulate containing sacubitril sodium salt of desired particle size. Both granulates are mixed. Filler, binder, disintegrant, glidant and lubricant are admixed in order to preparing the mixture ready for tableting. The said tableting mixture is compressed into tablets on a high speed tableting machine with round, lentil shaped tableting punches of 11 mm diameter.

| | | A | B | C | D |
|---|---|---|---|---|---|
| Valsartan disodium salt hydrate | active | 124.50 | 124.50 | 124.50 | 124.50 |
| Colloidal silicon dioxide | glidant | 2.00 | 2.00 | 2.00 | 2.00 |
| Talc | glidant | 3.00 | 3.00 | 3.00 | 3.00 |
| Magnesium stearate | lubricant | 6.00 | 6.00 | 6.00 | 6.00 |
| Total valsartan disodium salt hydrate containing granulate (mg) | | 135.50 | 135.50 | 135.50 | 135.50 |
| | | | | | |
| Sacubitril sodium salt | active | 102.40 | 102.40 | 102.40 | 102.40 |
| Colloidal silicon dioxide | glidant | 1.50 | 1.50 | 1.50 | 1.50 |
| Talc | glidant | 2.25 | 2.25 | 2.25 | 2.25 |
| Magnesium stearate | lubricant | 4.50 | 4.50 | 4.50 | 4.50 |
| Total sacubitril sodium salt containing granulate (mg) | | 110.65 | 110.65 | 110.65 | 110.65 |
| | | | | | |
| Microcrystalline cellulose | filler | 64.85 | | 54.85 | |
| Calcium hydrogen phosphate anhydrous | filler | | 64.85 | | 54.85 |
| Low-substituted hydroxypropyl cellulose | binder | 60.00 | 60.00 | 40.00 | 40.00 |
| Crospovidone | disintegrant | 20.00 | 20.00 | 40.00 | 40.00 |
| Talc | glidant | 1.00 | 1.00 | 1.00 | 1.00 |
| Magnesium stearate | lubricant | 8.00 | 8.00 | 8.00 | 8.00 |
| Total single layer tablet (mg) | | 400.00 | 400.00 | 400.00 | 400.00 |

The tableting process resulted in tablets with low tablet mass variability, which corresponds to high content uniformity of both active ingredients.

### Comparative example 2. Composition comprising valsartan disodium salt hydrate and sacubitril sodium salt prepared in the form of a bilayer tablet

Valsartan disodium salt hydrate, filler, binder, disintegrant, glidants and a lubricant are mixed, roller compacted and sieved thru 1 mm sieve. To the said sieved granulate, disintegrant, glidant and lubricant are admixed in order to prepare a mixture containing valsartan disodium salt hydrate ready for tableting.

Sacubitril sodium salt, filler, disintegrant glidants and a lubricant are roller compacted and sieved thru 1 mm sieve. To the said sieved granulate, disintegrant, glidant and a lubricant are admixed in order to prepare a mixture containing sacubitril sodium salt ready for tableting.

The said tableting mixtures are compressed into tablets on a high speed tableting machine with round, lentil shaped tableting punches of 12 mm diameter.

| | | A | B |
|---|---|---|---|
| Valsartan disodium salt hydrate | active | 124.50 | 124.50 |
| Microcrystalline cellulose | filler | 171.50 | 171.50 |
| Low-substituted hydroxypropyl cellulose | binder | 50.00 | 20.00 |
| Crospovidone | disintegrant | 20.00 | 50.00 |
| Colloidal silicon dioxide | glidant | 2.00 | 2.00 |
| Talc | glidant | 3.00 | 3.00 |
| Magnesium stearate | lubricant | 6.00 | 6.00 |
| Total valsartan disodium salt hydrate containing granulate (mg) | | 377.00 | 377.00 |
| Crospovidone | disintegrant | 16.00 | 2.00 |
| Talc | glidant | 1.00 | 3.00 |
| Magnesium stearate | lubricant | 6.00 | 6.00 |
| Total valsartan disodium salt hydrate containing layer (mg) | | 400.00 | 250.00 |
| | | | |
| Sacubitril sodium salt | active | 102.40 | 102.40 |
| Microcrystalline cellulose | filler | 156.80 | 166.80 |
| Crospovidone | disintegrant | 15.00 | 5.00 |
| Colloidal silicon dioxide | glidant | 1.50 | 1.50 |
| Talc | glidant | 2.40 | 2.40 |
| Magnesium stearate | lubricant | 4.50 | 4.50 |
| Total sacubitril sodium salt containing granulate (mg) | | 282.60 | 282.60 |
| Crospovidone | disintegrant | 12.00 | 12.00 |
| Colloidal silicon dioxide | glidant | 0.90 | 0.90 |
| Magnesium stearate | lubricant | 4.50 | 4.50 |
| Total sacubitril sodium salt containing layer (mg) | | 300.00 | 300.00 |
| | | | |
| Total bilayer tablet (mg) | | 700.00 | 700.00 |

During the tableting process, both valsartan disodium salt hydrate containing layer as well as sacubitril sodium salt layer exhibit poor material flow into the tableting die, resulting in a high tablet mass variability, which corresponds to low content uniformity of both active ingredients.

### Example 2A. Composition comprising valsartan disodium salt hydrate and sacubitril sodium salt as separate units prepared in the form of a bilayer layer tablet

Valsartan disodium salt hydrate, glidants and lubricant are mixed, roller compacted and sieved thru 1 mm sieve in order to obtain granulate containing valsartan disodium salt hydrate of desired particle size. The said granulate is admixed with filler, binder, disintegrant, glidant and lubricant in order to obtain valsartan disodium salt hydrate containing tableting mixture.

Sacubitril sodium salt, glidants and lubricant are mixed, roller compacted and sieved thru 1 mm sieve in order to obtain granulate containing sacubitril sodium salt of desired particle size. The said granulate is admixed with filler, disintegrant, glidant and lubricant in order to obtain sacubitril sodium salt containing tableting mixture.

The said tableting mixtures are compressed into tablets on a high speed tableting machine in bilayer tableting mode with round, lentil shaped tableting punches of 11 mm diameter.

| | | A | B |
|---|---|---|---|
| Valsartan disodium salt hydrate | active | 124.50 | 124.50 |
| Colloidal silicon dioxide | glidant | 2.00 | 2.00 |
| Talc | glidant | 3.00 | 3.00 |
| Magnesium stearate | lubricant | 6.00 | 6.00 |
| Total valsartan disodium salt hydrate containing granulate (mg) | | 135.50 | 135.50 |
| Microcrystalline cellulose | filler | 45.00 | 65.00 |
| Low-substituted hydroxypropyl cellulose | binder | 60.00 | 40.00 |
| Crospovidone | disintegrant | 5.00 | 5.00 |
| Talc | glidant | 0.50 | 0.50 |
| Magnesium stearate | lubricant | 4.00 | 4.00 |
| Total valsartan disodium salt hydrate containing layer (mg) | | 250.00 | 250.00 |
| | | | |
| Sacubitril sodium salt | active | 102.40 | 102.40 |
| Colloidal silicon dioxide | glidant | 1.50 | 1.50 |
| Talc | glidant | 2.25 | 2.25 |
| Magnesium stearate | lubricant | 4.50 | 4.50 |
| Total sacubitril sodium salt containing granulate (mg) | | 110.65 | 110.65 |
| Microcrystalline cellulose | filler | 121.45 | |
| Calcium hydrogen phosphate anhydrous | filler | | 121.45 |
| Crospovidone | disintegrant | 12.50 | 12.50 |
| Talc | glidant | 0.90 | 0.90 |
| Magnesium stearate | lubricant | 4.50 | 4.50 |
| Total sacubitril sodium salt containing layer (mg) | | 250.00 | 250.00 |
| | | | |
| Total bilayer tablet (mg) | | 500.00 | 500.00 |

The tableting process resulted in tablets with low tablet mass variability, which corresponds to high content uniformity of both active ingredients.

### Dissolution measurement method

Dissolution rate of prepared prototypes were characterized using USP 2 (paddle apparatus). Measurements were performed using 900 mL of solution at constant pH 2.0 and ionic strength of 100 mM adjusted by sodium chloride and hydrochloric acid. Agitation rate was kept constant at 75 RPM for 45 min which was then increased to 150 RPM for the last 15 minutes of the measurement. Samples were collected into vials after 2, 5, 10, 15, 20, 25, 30, 45, 50 and 60 minutes. Dissolved amount was then determined using liquid chromatography.

## Claims

1. A composition containing a combination of
valsartan disodium salt hydrate or valsartan free acid, and
sacubitril sodium salt,
***characterized in that*** the said composition contains
- particles containing valsartan disodium salt hydrate or valsartan free acid, at least one lubricant and optionally at least one glidant, but not containing fillers, binders, nor disintegrants; and
- particles containing sacubitril sodium salt, at least one lubricant and optionally at least one glidant, but not containing fillers, binders, nor disintegrants; and
- extra-particular phase containing at least one filler, and optionally at least one binder and optionally at least one disintegrant.

2. The composition according to claim 1, wherein the particles containing valsartan disodium salt hydrate or valsartan free acid contain the at least one lubricant in the total amount of 0.5 w/w% or more, preferably in the amount of 1 w/w% or more.

3. The composition according to claim 1 or 2, wherein the particles containing sacubitril sodium salt contain the at least one lubricant in the total amount of 0.5 w/w% or more, preferably in the amount of 1 w/w% or more.

4. The composition according to any one of the preceding claims, wherein the composition comprising valsartan disodium salt hydrate or valsartan free acid and sacubitril sodium salt, respectively, as separate particles contains a further extraparticular amount of at least one lubricant in the total amount of 0.5 w/w% or more, preferably in the amount of 1 w/w% or more.

5. The composition according to any one of the preceding claims, wherein the extra-particular phase contains at least one disintegrant.

6. The composition according to any one of the preceding claims, wherein
- the valsartan disodium salt hydrate is present at least partially in the form defined as Form I, which is **characterized by** an X-ray powder diffraction pattern having peaks at about 4.6; 9.3; 12.8; 15.5; 18.4 and 22.3 degrees two theta ± 0.2 degrees two theta; and/or
- the sacubitril sodium salt is present at least partially in the form defined as Form I, which is
**characterized by** an X-ray powder diffraction pattern having peaks at about 3.1; 6.2; 11.9; 16.4; 18.3 and 19.9 degrees two theta ± 0.2 degrees two theta.

7. The composition according to any one of the preceding claims, wherein
- the lubricant is selected from magnesium stearate, talc, calcium stearate, stearic acid, hydrogenated vegetable oil, glyceryl behenate, sodium stearyl fumarate, polyethylene glycol, sodium lauryl sulphate, isoleucine and sodium benzoate and mixtures thereof; and/or
- the glidant, if present, is selected from colloidal silicon dioxide, anhydrous colloidal silica, magnesium oxide, magnesium silicate, magnesium trisilicate, talc, calcium silicate and tribasic calcium phosphate and mixtures thereof.

8. A tablet comprising the composition according to any one of the preceding claims, wherein the tablet is a single-layer tablet.

9. A tablet comprising the composition according to any one of claims 1 to 7, wherein the tablet is a multi-layer tablet, wherein the valsartan disodium salt hydrate is present in a different layer than sacubitril sodium salt.

10. The composition according to any one of the claims 1 to 7 or the tablet according to claim 8 or 9, which contains:
- particles containing valsartan disodium salt hydrate or valsartan free acid, at least one lubricant and optionally at least one glidant, but not containing fillers, binders, nor disintegrants; and
- particles containing sacubitril sodium salt, at least one lubricant and optionally at least one glidant, but not containing fillers, binders, nor disintegrants; and
- extra-particular phase containing at least one filler, optionally at least one binder and optionally at least one disintegrant,
wherein
- the filler is selected from oligosaccharides, monosaccharides, sugar alcohols, hydroxypropyl cellulose, microcrystalline cellulose, silicified microcrystalline cellulose, calcium hydrogen phosphate anhydrous, calcium hydrogen phosphate dihydrate, and mixtures thereof; and/or
- the glidant is selected from colloidal silicon dioxide, anhydrous colloidal silica, magnesium oxide, magnesium silicate, magnesium trisilicate, talc, calcium silicate and tribasic calcium phosphate and mixtures thereof;
- the lubricant is selected from magnesium stearate, talc, calcium stearate, stearic acid, hydrogenated vegetable oil, glyceryl behenate, sodium stearyl fumarate, polyethylene glycol, sodium lauryl sulphate, isoleucine and sodium benzoate and mixtures thereof;
- the binder, if present, is selected from pregelatinized starch, polymethacrylates, copovidone, povidone, maltose, starch, cellulose powder, crystalline cellulose, microcrystalline cellulose, silicified microcrystalline cellulose, cellulose derivatives such as hydroxymethylcellulose, hydroxyethylcellulose, hydroxypropyl cellulose, low-substituted hydroxypropyl cellulose and hydroxypropylmethylcellulose, and mixtures thereof; and/or
- the disintegrant, if present is selected from croscarmellose sodium, crospovidone, hydroxypropyl starch, low-substituted hydroxypropyl cellulose, sodium starch glycolate and mixtures thereof.

11. The composition according to any one of the claims 1 to 7 or the tablet according to claim 8 or 9, which contains:
- particles containing valsartan disodium salt hydrate or valsartan free acid, at least one lubricant and at least one glidant, but not containing fillers, binders, nor disintegrants; and
- particles containing sacubitril sodium salt, at least one lubricant and at least one glidant, but not containing fillers, binders, nor disintegrants; and
- extra-particular phase containing at least one filler, at least one binder and at least one disintegrant,
wherein
- the filler is selected from oligosaccharides, monosaccharides, sugar alcohols, hydroxypropyl cellulose, microcrystalline cellulose, silicified microcrystalline cellulose, calcium hydrogen phosphate anhydrous, calcium hydrogen phosphate dihydrate, and mixtures thereof; and/or
- the binder is selected from pregelatinized starch, polymethacrylates, copovidone, povidone, maltose, starch, cellulose powder, crystalline cellulose, microcrystalline cellulose, silicified microcrystalline cellulose, cellulose derivatives such as hydroxymethylcellulose, hydroxyethylcellulose, hydroxypropyl cellulose, low-substituted hydroxypropyl cellulose and hydroxypropylmethylcellulose, and mixtures thereof; and/or
- the disintegrant is selected from croscarmellose sodium, crospovidone, hydroxypropyl starch, low-substituted hydroxypropyl cellulose, sodium starch glycolate and mixtures thereof; and/or
- the lubricant is selected from magnesium stearate, talc, calcium stearate, stearic acid, hydrogenated vegetable oil, glyceryl behenate, sodium stearyl fumarate, polyethylene glycol, sodium lauryl sulphate, isoleucine and sodium benzoate and mixtures thereof; and/or

12. The composition or the tablet according to claim 11, wherein
- the filler is selected from mannitol, lactose anhydrous, microcrystalline cellulose, silicified microcrystalline cellulose, calcium hydrogen phosphate anhydrous, hydroxypropyl cellulose and mixtures thereof; and/or
- the binder is selected from microcrystalline cellulose and low-substituted hydroxypropyl cellulose and mixtures thereof; and/or
- the disintegrant is selected from crospovidone and low-substituted hydroxypropyl cellulose and mixtures thereof; and/or
- the lubricant is magnesium stearate and/or talc; and/or
- the glidant is selected from colloidal silicon dioxide, anhydrous colloidal silica, talc, and mixtures thereof.

13. Method of preparation of the composition according to any one of the claims 1-7, 8-12, or of the tablet according to any one of the claims 8-12, comprising the steps in which
- mixture of valsartan disodium salt hydrate or valsartan free acid with at least one lubricant and optionally with at least one glidant, but without fillers, binders or disintegrants, is processed by dry manufacturing process, preferably roller compacted or slugged;
- mixture of sacubitril sodium salt with at least one lubricant and optionally with at least one glidant, but without fillers, binders or disintegrants, is processed by dry manufacturing process, preferably roller compacted or slugged.

14. Method of preparation of the single-layer tablet according to any one of the claims 8, 10-12, which comprises the following steps:
- mixing of valsartan disodium salt hydrate or valsartan free acid with at least one lubricant and optionally with at least one glidant;
- roller compaction or slugging of the mixture containing valsartan disodium salt hydrate or valsartan free acid into compactate or slugs;
- sieving or milling the compactate or slugs into particles containing valsartan disodium salt hydrate valsartan free acid;
- . mixing of sacubitril sodium salt with at least one lubricant and optionally with at least one glidant;
- roller compaction or slugging of the mixture containing sacubitril sodium salt into compactate or slugs;
- sieving or milling the compactate or slugs into particles containing sacubitril sodium salt;
- mixing of the particles containing valsartan disodium salt hydrate or valsartan free acid and the particles containing sacubitril sodium salt;
- admixing further excipients to prepare a mixture ready for tableting;
- compressing the said mixture into a tablet.

15. Method of preparation of the multi-layer tablet according to any one of the claims 9-12, which comprises the following steps:
- mixing of valsartan disodium salt hydrate or valsartan free acid with at least one lubricant and optionally with at least one glidant;
- roller compaction or slugging of the mixture containing valsartan disodium salt hydrate or valsartan free acid into compactate or slugs;
- sieving or milling the compactate or slugs into particles containing valsartan disodium salt hydrate or valsartan free acid;
- admixing optional further excipients in order to prepare a mixture containing valsartan disodium salt hydrate or valsartan free acid ready for tableting;
- mixing of sacubitril sodium salt with at least one lubricant and optionally with at least one glidant;
- roller compaction or slugging of the mixture containing sacubitril sodium salt into compactate or slugs;
- sieving or milling the compactate or slugs into particles containing sacubitril sodium salt;
- admixing optional further excipients in order to prepare a mixture containing sacubitril sodium salt ready for tableting;
- optionally preparing a separation-layer mixture not containing any active ingredient;
- compressing the said valsartan disodium salt hydrate or valsartan free acid mixture, the sacubitril sodium salt mixture and the optional separation mixture not containing any active ingredient into a multilayer tablet.

## Patentansprüche

1. Eine Zusammensetzung, die eine Kombination von
Valsartan-Dinatriumsalz-Hydrat oder Valsartan in Form von freier Säure und
Sacubitril-Natriumsalz,
**dadurch gekennzeichnet, dass** die Zusammensetzung enthält
- Partikel, die Valsartan-Dinatriumsalz-Hydrat oder Valsartan in Form von freier Säure, mindestens ein Schmiermittel und gegebenenfalls mindestens ein Gleitmittel enthalten, jedoch keine Füllstoffe, Bindemittel oder Sprengmittel enthalten; und
- Partikel, die Sacubitril-Natriumsalz, mindestens ein Schmiermittel und gegebenenfalls mindestens ein Gleitmittel enthalten, jedoch keine Füllstoffe, Bindemittel oder Sprengmittel enthalten; und
- extrapartikuläre Phase, enthaltend mindestens einen Füllstoff und gegebenenfalls mindestens ein Bindemittel und optional mindestens ein Sprengmittel.

2. Zusammensetzung nach Anspruch 1, wobei die Partikel, die Valsartan-Dinatriumsalz-Hydrat oder Valsartan in Form von freier Säure enthalten, mindestens ein Schmiermittel in einer Gesamtmenge von 0,5 Gew.-% oder mehr, vorzugsweise von 1 Gew.-% oder mehr, enthalten.

3. Zusammensetzung nach Anspruch 1 oder 2, wobei die Sacubitril-Natriumsalz enthaltenden Partikel mindestens ein Schmiermittel in einer Gesamtmenge von 0,5 Gew.-% oder mehr, vorzugsweise von 1 Gew.-% oder mehr, enthalten.

4. Zusammensetzung nach einem der vorhergehenden Ansprüche, wobei die Zusammensetzung, die Valsartan-Dinatriumsalz-Hydrat oder Valsartan in Form von freier Säure bzw. Sacubitril-Natriumsalz als separate Partikel umfasst, und eine weitere extrapartikuläre Menge mindestens eines Gleitmittels in der Gesamtmenge von 0,5 Gew.-% oder mehr, vorzugsweise von 1 Gew.-% oder mehr, enthält.

5. Zusammensetzung nach einem der vorhergehenden Ansprüche, wobei die extrapartikuläre Phase mindestens ein Sprengmittel enthält.

6. Zusammensetzung nach einem der vorhergehenden Ansprüche, wobei
- das Valsartan-Dinatriumsalz-Hydrat liegt zumindest teilweise in der als Form I definierten Form vor, die durch ein Röntgenpulverbeugungsmuster mit Peaks bei etwa 4,6; 9,3; 12,8; 15,5; 18,4 und 22,3 Grad zwei Theta ± 0,2 Grad zwei Theta gekennzeichnet ist; und/oder
- das Sacubitril-Natriumsalz liegt zumindest teilweise in der als Form I definierten Form vor, die durch ein Röntgenpulverbeugungsmuster mit Peaks bei etwa 3,1; 6,2; 11,9; 16,4; 18,3 und 19,9 Grad zwei Theta ± 0,2 Grad zwei Theta gekennzeichnet ist.

7. Zusammensetzung nach einem der vorhergehenden Ansprüche, wobei
- das Schmiermittel ausgewählt ist aus Magnesiumstearat, Talkum, Calciumstearat, Stearinsäure, hydriertem Pflanzenöl, Glycerylbehenat, Natriumstearylfumarat, Polyethylenglycol, Natriumlaurylsulfat, Isoleucin und Natriumbenzoat und Mischungen davon; und/oder
- das Gleitmittel, falls vorhanden, ausgewählt ist aus kolloidalem Siliciumdioxid, wasserfreiem kolloidalem Siliciumdioxid, Magnesiumoxid, Magnesiumsilicat, Magnesiumtrisilicat, Talkum, Calciumsilicat und tribasischem Calciumphosphat und Mischungen davon.

8. Tablette, umfassend die Zusammensetzung nach einem der vorhergehenden Ansprüche, wobei die Tablette eine einschichtige Tablette ist.

9. Tablette, umfassend die Zusammensetzung nach einem der Ansprüche 1 bis 7, wobei die Tablette eine Mehrschichttablette ist, wobei das Valsartan-Dinatriumsalz-Hydrat in einer anderen Schicht als Sacubitril-Natriumsalz vorliegt.

10. Zusammensetzung nach einem der Ansprüche 1 bis 7 oder Tablette nach Anspruch 8 oder 9, die enthält:
- Partikel, die Valsartan-Dinatriumsalz-Hydrat oder Valsartan in Form von freier Säure, mindestens ein Schmiermittel und gegebenenfalls mindestens ein Gleitmittel enthalten, jedoch keine Füllstoffe, Bindemittel oder Sprengmittel enthalten; und
- Partikel, die Sacubitril-Natriumsalz, mindestens ein Schmiermittel und gegebenenfalls mindestens ein Gleitmittel enthalten, jedoch keine Füllstoffe, Bindemittel oder Sprengmittel enthalten; und
- extrapartikuläre Phase enthaltend mindestens einen Füllstoff, gegebenenfalls mindestens ein Bindemittel und gegebenenfalls mindestens ein Sprengmittel,
worin
- der Füllstoff ist ausgewählt aus Oligosacchariden, Monosacchariden, Zuckeralkoholen, Hydroxypropylcellulose, mikrokristalliner Cellulose, verkieselter mikrokristalliner Cellulose, wasserfreiem Calciumhydrogenphosphat, Calciumhydrogenphosphat-Dihydrat und Mischungen davon; und/oder
- das Gleitmittel ist ausgewählt aus kolloidalem Siliciumdioxid, wasserfreiem kolloidalem Siliciumdioxid, Magnesiumoxid, Magnesiumsilicat, Magnesiumtrisilicat, Talkum, Calciumsilicat und tribasischem Calciumphosphat und Mischungen davon;
- das Schmiermittel ausgewählt ist aus Magnesiumstearat, Talkum, Calciumstearat, Stearinsäure, hydriertem Pflanzenöl, Glycerylbehenat, Natriumstearylfumarat, Polyethylenglycol, Natriumlaurylsulfat, Isoleucin und Natriumbenzoat und Mischungen davon;
- das Bindemittel, falls vorhanden, ausgewählt ist aus vorgelatinierter Stärke, Polymethacrylaten, Copovidon, Povidon, Maltose, Stärke, Cellulosepulver, kristalliner Cellulose, mikrokristalliner Cellulose, verkieselter mikrokristalliner Cellulose, Cellulosederivaten wie Hydroxymethylcellulose, Hydroxyethylcellulose, Hydroxypropylcellulose, niedrig substituiert Hydroxypropylcellulose und Hydroxypropylmethylcellulose und Mischungen davon; und/oder
- das Sprengmittel, falls vorhanden, ausgewählt ist aus Croscarmellose-Natrium, Crospovidon, Hydroxypropylstärke, niedrigsubstituierter Hydroxypropylcellulose, Natriumstärkeglycolat und Mischungen davon.

11. Zusammensetzung nach einem der Ansprüche 1 bis 7 oder Tablette nach Anspruch 8 oder 9, die enthält:
- Partikel, die Valsartan-Dinatriumsalz-Hydrat oder Valsartan in Form von freier Säure, mindestens ein Schmiermittel und mindestens ein Gleitmittel enthalten, jedoch keine Füllstoffe, Bindemittel oder Sprengmittel enthalten; und
- Partikel, die Sacubitril-Natriumsalz, mindestens ein Schmiermittel und mindestens ein Gleitmittel enthalten, jedoch keine Füllstoffe, Bindemittel oder Sprengmittel enthalten; und
- extrapartikuläre Phase enthaltend mindestens einen Füllstoff, mindestens ein Bindemittel und mindestens ein Sprengmittel,
worin
- der Füllstoff ist ausgewählt aus Oligosacchariden, Monosacchariden, Zuckeralkoholen, Hydroxypropylcellulose, mikrokristalliner Cellulose, verkieselter mikrokristalliner Cellulose, wasserfreiem Calciumhydrogenphosphat, Calciumhydrogenphosphat-Dihydrat und Mischungen davon; und/oder
- das Bindemittel ausgewählt ist aus vorgelatinierter Stärke, Polymethacrylaten, Copovidon, Povidon, Maltose, Stärke, Cellulosepulver, kristalliner Cellulose, mikrokristalliner Cellulose, verkieselter mikrokristalliner Cellulose, Cellulosederivaten wie Hydroxymethylcellulose, Hydroxyethylcellulose, Hydroxypropylcellulose, niedrig substituierte Hydroxypropylcellulose und Hydroxypropylmethylcellulose , und Mischungen davon; und/oder
- das Sprengmittel ausgewählt ist aus Croscarmellose-Natrium, Crospovidon, Hydroxypropylstärke, niedrigsubstituierter Hydroxypropylcellulose, Natriumstärkeglycolat und Mischungen davon; und/oder
- das Schmiermittel ausgewählt ist aus Magnesiumstearat, Talkum, Calciumstearat, Stearinsäure, hydriertem Pflanzenöl, Glycerylbehenat, Natriumstearylfumarat, Polyethylenglycol, Natriumlaurylsulfat, Isoleucin und Natriumbenzoat und Mischungen davon.

12. Zusammensetzung oder Tablette nach Anspruch 11, wobei
- der Füllstoff ist ausgewählt aus Mannit, wasserfreier Lactose, mikrokristalliner Cellulose, verkieselter mikrokristalliner Cellulose, wasserfreiem Calciumhydrogenphosphat, Hydroxypropylcellulose und Mischungen davon; und/oder
- das Bindemittel ist ausgewählt aus mikrokristalliner Cellulose und niedrig substituierter Hydroxypropylcellulose und Mischungen davon; und/oder
- das Sprengmittel ist ausgewählt aus Crospovidon und niedrig substituierter Hydroxypropylcellulose und Mischungen davon; und/oder
- das Schmiermittel Magnesiumstearat und/oder Talkum ist; und/oder
- das Gleitmittel ist ausgewählt aus kolloidalem Siliciumdioxid, wasserfreiem kolloidalem Siliciumdioxid, Talkum und Mischungen davon.

13. Verfahren zur Herstellung der Zusammensetzung nach einem der Ansprüche 1-7, 8-12 oder der Tablette nach einem der Ansprüche 8-12, umfassend die Schritte, bei denen
- Mischung aus Valsartan-Dinatriumsalz-Hydrat oder Valsartan in Form von freier Säure mit mindestens einem Schmiermittel und gegebenenfalls mit mindestens einem Gleitmittel, jedoch ohne Füllstoffe, Bindemittel oder Sprengmittel, wird im Trockenherstellungsverfahren verarbeitet, vorzugsweise walzenkompaktiert oder geschlagen;
- Mischung von Sacubitril-Natriumsalz mit mindestens einem Schmiermittel und gegebenenfalls mit mindestens einem Gleitmittel, jedoch ohne Füllstoffe, Bindemittel oder Sprengmittel, wird im Trockenherstellungsverfahren verarbeitet, vorzugsweise walzenkompaktiert oder geschlagen.

14. Verfahren zur Herstellung der einschichtigen Tablette nach einem der Ansprüche 8, 10-12, welches die folgenden Schritte umfasst:
- Mischen von Valsartan-Dinatriumsalz-Hydrat oder Valsartan in Form von freier Säure mit mindestens einem Schmiermittel und gegebenenfalls mit mindestens einem Gleitmittel;
- Walzenkompaktieren oder schlagen der Mischung, die Valsartan-Dinatriumsalz-Hydrat oder Valsartan in Form von freier Säure enthält, zu Kompaktat oder Slugs;
- Sieben oder Mahlen des Kompaktats oder der Slugs zu Partikeln, die Valsartan-Dinatriumsalz-Hydrat oder Valsartan in Form von freier Säure enthalten;
- Mischen von Sacubitril-Natriumsalz mit mindestens einem Schmiermittel und gegebenenfalls mit mindestens einem Gleitmittel;
- Walzenkompaktiereng oder schlagen der Sacubitril-Natriumsalz enthaltenden Mischung zu Kompaktaten oder Slugs;
- Sieben oder Mahlen des Kompaktats oder der Slugs zu Partikeln, die Sacubitril-Natriumsalz enthalten;
- Mischen der Partikel, die Valsartan-Dinatriumsalz-Hydrat oder Valsartan in Form von freier Säure enthalten, und der Partikel, die Sacubitril-Natriumsalz enthalten;
- Zumischen weiterer Hilfsstoffe, um eine tablettierfähige Mischung herzustellen;
- Komprimieren der Mischung zu einer Tablette.

15. Verfahren zur Herstellung der Mehrschichttablette nach einem der Ansprüche 9-12, welches die folgenden Schritte umfasst:
- Mischen von Valsartan-Dinatriumsalz-Hydrat oder Valsartan in Form von freier Säure mit mindestens einem Schmiermittel und gegebenenfalls mit mindestens einem Gleitmittel;
- Walzenkompaktieren oder schlagen der Mischung, die Valsartan-Dinatriumsalz-Hydrat oder Valsartan in Form von freier Säure enthält, zu Kompaktat oder Slugs;
- Sieben oder Mahlen des Kompaktats oder der Slugs zu Partikeln, die Valsartan-Dinatriumsalz-Hydrat oder Valsartan in Form von freier Säure enthalten;
- Zuimischen gegebenenfalls weiterer Hilfsstoffe, um eine tablettierungsfertige Mischung herzustellen, die Valsartan-Dinatriumsalz-Hydrat oder Valsartan in Form von freier Säure enthält;
- Mischen von Sacubitril-Natriumsalz mit mindestens einem Schmiermittel und gegebenenfalls mit mindestens einem Gleitmittel;
- Walzenkompaktieren oder schlagen der Sacubitril-Natriumsalz enthaltenden Mischung zu Kompaktaten oder Slugs;
- Sieben oder Mahlen des Kompaktats oder der Slugs zu Partikeln, die Sacubitril-Natriumsalz enthalten;
- Zumischen gegebenenfalls weiterer Hilfsstoffe, um eine tablettierfertige Mischung enthaltend Sacubitril-Natriumsalz herzustellen;
- gegebenenfalls Herstellen einer Trennschichtmischung, die keinen Wirkstoff enthält;
- Komprimieren der Mischung des Valsartan-Dinatriumsalz-Hydrats oder des Valsartans in Form von freier Säure, der Sacubitril-Natriumsalz-Mischung und der optionalen Trennmischung, die keinen Wirkstoff enthält, zu einer mehrschichtigen Tablette.

## Revendications

1. Une composition contenant une combinaison de
hydrate de sel disodique de valsartan ou valsartan sous forme d'acide libre, et
sel de sodium de sacubitril,
**caractérisé en ce que** ladite composition contient
- des particules contenant de l'hydrate de sel disodique valsartan ou du valsartan sous forme d'acide libre, au moins un lubrifiant et éventuellement au moins un glissant, mais ne contenant pas de charges, de liants, ni de délitants; et
- des particules contenant du sel de sodium de sacubitril, au moins un lubrifiant et éventuellement au moins un glissant, mais ne contenant pas de charges, de liants, ni de délitants; et
- phase extra-particulaire contenant au moins une charge, et éventuellement au moins un liant et éventuellement au moins un délitant.

2. Composition selon la revendication 1, où les particules contenant de l'hydrate de sel disodique de valsartan ou du valsartan sous forme d'acide libre contiennent ledit au moins un lubrifiant en une quantité totale de 0,5 % en poids ou plus, de préférence en une quantité de 1 % en poids ou plus.

3. Composition selon la revendication 1 ou 2, où les particules contenant du sel de sodium de sacubitril contiennent ledit au moins un lubrifiant en une quantité totale de 0,5 % en poids ou plus, de préférence en une quantité de 1 % en poids ou plus.

4. Composition selon l'une quelconque des revendications précédentes, où la composition comprenant de l'hydrate de sel disodique de valsartan ou du valsartan sous forme d'acide libre et du sel de sodium de sacubitril, respectivement, sous forme de particules séparées, contient une quantité extra-particulaire supplémentaire d'au moins un lubrifiant dans la quantité totale de 0,5 % en poids ou plus, de préférence en une quantité de 1 % en poids ou plus.

5. Composition selon l'une quelconque des revendications précédentes, où la phase extra-particulaire contient au moins un délitant.

6. Composition selon l'une quelconque des revendications précédentes, où
- l'hydrate de sel disodique de valsartan est présent au moins partiellement sous la forme définie comme la Forme I, qui est **caractérisée par** un diagramme de diffraction des rayons X sur poudre ayant des pics à environ 4,6; 9.3; 12,8; 15,5; 18,4 et 22,3 degrés deux thêta ± 0,2 degrés deux thêta ; et/ou
- le sel de sodium du sacubitril est présent au moins partiellement sous la forme définie comme la Forme I, qui est **caractérisé par** un diagramme de diffraction des rayons X sur poudre ayant des pics à environ 3,1; 6,2; 11,9; 16,.4; 18,3 et 19,9 degrés deux thêta ± 0,2 degrés deux thêta.

7. Composition selon l'une quelconque des revendications précédentes, où
- le lubrifiant est choisi parmi le stéarate de magnésium, le talc, le stéarate de calcium, l'acide stéarique, l'huile végétale hydrogénée, le béhénate de glycéryle, le stéarylfumarate de sodium, le polyéthylène glycol, le lauryl sulfate de sodium, l'isoleucine et le benzoate de sodium et leurs mélanges ; et/ou
- le glissant, s'il est présent, est choisi parmi le dioxyde de silicium colloïdal, la silice colloïdale anhydre, l'oxyde de magnésium, le silicate de magnésium, le trisilicate de magnésium, le talc, le silicate de calcium et le phosphate de calcium tribasique et leurs mélanges.

8. Comprimé comprenant la composition selon l'une quelconque des revendications précédentes, où le comprimé est un comprimé monocouche.

9. Comprimé comprenant la composition selon l'une quelconque des revendications 1 à 7, où le comprimé est un comprimé multicouche, dans lequel l'hydrate de sel disodique de valsartan est présent dans une couche différente du sel de sodium de sacubitril.

10. Composition selon l'une quelconque des revendications 1 à 7 ou comprimé selon la revendication 8 ou 9, qui contient:
- des particules contenant de l'hydrate de sel disodique de valsartan ou du valsartan sous forme d'acide libre, au moins un lubrifiant et éventuellement au moins un glissant, mais ne contenant pas de charges, de liants, ni de délitants; et
- des particules contenant du sel de sodium de sacubitril, au moins un lubrifiant et éventuellement au moins un glissant, mais ne contenant pas de charges, de liants, ni de délitants; et
- phase extra-particulaire contenant au moins une charge, éventuellement au moins un liant et éventuellement au moins un délitant,
où
- la charge est choisie parmi les oligosaccharides, les monosaccharides, les alcools de sucre, l'hydroxypropylcellulose, la cellulose microcristalline, la cellulose microcristalline silicifiée, l'hydrogénophosphate de calcium anhydre, l'hydrogénophosphate de calcium dihydraté, et leurs mélanges; et/ou
- le glissant est choisi parmi le dioxyde de silicium colloïdal, la silice colloïdale anhydre, l'oxyde de magnésium, le silicate de magnésium, le trisilicate de magnésium, le talc, le silicate de calcium et le phosphate de calcium tribasique et leurs mélanges;
- le lubrifiant est choisi parmi le stéarate de magnésium, le talc, le stéarate de calcium, l'acide stéarique, l'huile végétale hydrogénée, le béhénate de glycéryle, le stéarylfumarate de sodium, le polyéthylène glycol, le lauryl sulfate de sodium, l'isoleucine et le benzoate de sodium et leurs mélanges;
- le liant, s'il est présent, est choisi parmi l'amidon prégélatinisé, les polyméthacrylates, la copovidone, la povidone, le maltose, l'amidon, la cellulose en poudre, la cellulose cristalline, la cellulose microcristalline, la cellulose microcristalline silicifiée, les dérivés cellulosiques tels que l'hydroxyméthylcellulose, l'hydroxyéthylcellulose, l'hydroxypropylcellulose, l'hydroxypropylcellulose faiblement substituée, et l'hydroxypropylméthylcellulose, et leurs mélanges ; et/ou
- le délitant, s'il est présent, est choisi parmi la croscarmellose sodique, la crospovidone, l'hydroxypropylamidon, l'hydroxypropylcellulose faiblement substituée, le glycolate d'amidon sodique et leurs mélanges.

11. Composition selon l'une quelconque des revendications 1 à 7 ou comprimé selon la revendication 8 ou 9, qui contient:
- des particules contenant de l'hydrate de sel disodique de valsartan ou du valsartan sous forme d'acide libre, au moins un lubrifiant et éventuellement au moins un glissant, mais ne contenant pas de charges, de liants, ni de délitants; et
- des particules contenant du sel de sodium de sacubitril, au moins un lubrifiant et éventuellement au moins un glissant, mais ne contenant pas de charges, de liants, ni de délitants; et
- phase extra-particulaire contenant au moins une charge, au moins un liant et au moins un délitant,
où
- la charge est choisie parmi les oligosaccharides, les monosaccharides, les alcools de sucre, l'hydroxypropylcellulose, la cellulose microcristalline, la cellulose microcristalline silicifiée, l'hydrogénophosphate de calcium anhydre, l'hydrogénophosphate de calcium dihydraté, et leurs mélanges; et/ou
- le liant est choisi parmi l'amidon prégélatinisé, les polyméthacrylates, la copovidone, la povidone, le maltose, l'amidon, la poudre de cellulose, la cellulose cristalline, la cellulose microcristalline, la cellulose microcristalline silicifiée, les dérivés cellulosiques tels que l'hydroxyméthylcellulose, l'hydroxyéthylcellulose, l'hydroxypropylcellulose, l'hydroxypropylcellulose faiblement substituée et l'hydroxypropylméthylcellulose, et leurs mélanges; et/ou
- le délitant est choisi parmi la croscarmellose sodique, la crospovidone, l'hydroxypropylamidon, l'hydroxypropylcellulose faiblement substituée, le glycolate d'amidon sodique et leurs mélanges ; et/ou
- le lubrifiant est choisi parmi le stéarate de magnésium, le talc, le stéarate de calcium, l'acide stéarique, l'huile végétale hydrogénée, le béhénate de glycéryle, le stéarylfumarate de sodium, le polyéthylène glycol, le lauryl sulfate de sodium, l'isoleucine et le benzoate de sodium et leurs mélanges.

12. Composition ou comprimé selon la revendication 11, où
- la charge est choisie parmi le mannitol, le lactose anhydre, la cellulose microcristalline, la cellulose microcristalline silicifiée, l'hydrogénophosphate de calcium anhydre, l'hydroxypropylcellulose et leurs mélanges; et/ou
- le liant est choisi parmi la cellulose microcristalline et l'hydroxypropylcellulose faiblement substituée et leurs mélanges; et/ou
- le délitant est choisi parmi la crospovidone et l'hydroxypropylcellulose faiblement substituée et leurs mélanges; et/ou
- le lubrifiant est du stéarate de magnésium et/ou du talc; et/ou
- le glissant est choisi parmi le dioxyde de silicium colloïdal, la silice colloïdale anhydre, le talc, et leurs mélanges.

13. Procédé de préparation de la composition selon l'une quelconque des revendications 1-7, 8-12, ou du comprimé selon l'une quelconque des revendications 8-12, comprenant les étapes dans lesquelles
- le mélange d'hydrate de sel disodique de valsartan ou de valsartan sous forme d'acide libre avec au moins un lubrifiant et éventuellement avec au moins un glissant, mais sans charges, liants ou délitants, est traité par procédé de fabrication à sec, de préférence compacté au rouleau ou maculé;
- le mélange de sel de sodium de sacubitril avec au moins un lubrifiant et éventuellement avec au moins un glissant, mais sans charges, liants ou délitants, est traité par procédé de fabrication à sec, de préférence compacté au rouleau ou maculé.

14. Procédé de préparation du comprimé monocouche selon l'une quelconque des revendications 8, 10-12, comprenant les étapes suivantes :
- mélange d'hydrate de sel disodique de valsartan ou de valsartan sous forme d'acide libre avec au moins un lubrifiant et éventuellement avec au moins un glissant ;
- compactage au rouleau ou maculage du mélange contenant d'hydrate de sel disodique de valsartan ou du valsartan sous forme d'acide libre en compactat ou en granulés;
- tamisage ou broyage du compactat ou des granulés en particules contenant d'hydrate de sel disodique de valsartan ou du valsartan sous forme d'acide libre;
- mélange de sel de sodium de sacubitril avec au moins un lubrifiant et éventuellement avec au moins un glissant;
- compactage au rouleau ou broyage du mélange contenant le sel de sodium de sacubitril en compactat ou en granulés;
- tamisage ou broyage de compactat ou des granulés en particules contenant du sel de sodium de sacubitril;
- mélange des particules contenant d'hydrate de sel disodique de valsartan ou du valsartan sous forme d'acide libre et des particules contenant du sel de sodium de sacubitril;
- mélange d'autres excipients pour préparer un mélange prêt à être comprimé;
- comprimer ledit mélange en un comprimé.

15. Procédé de préparation du comprimé multicouche selon l'une quelconque des revendications 9 à 12, comprenant les étapes suivantes:
- mélange d'hydrate de sel disodique de valsartan ou de valsartan sous forme d'acide libre avec au moins un lubrifiant et éventuellement avec au moins un glissant;
- compactage au rouleau ou maculage du mélange contenant d'hydrate de sel disodique de valsartan ou du valsartan sous forme d'acide libre en compactat ou en granulés;
- tamisage ou broyage du compactat ou des granulés en particules contenant d'hydrate de sel disodique de valsartan ou du valsartan sous forme d'acide libre;
- mélange d'autres excipients facultatifs afin de préparer un mélange contenant d'hydrate de sel disodique de valsartan ou du valsartan sous forme d'acide libre prêt à être comprimé;
- mélange de sel de sodium de sacubitril avec au moins un lubrifiant et éventuellement avec au moins un glissant;
- compactage au rouleau ou broyage du mélange contenant le sel de sodium de sacubitril en compactat ou en granulés;
- tamisage ou broyage du compactat ou des granulés en particules contenant du sel de sodium de sacubitril;
- mélange d'autres excipients facultatifs afin de préparer un mélange contenant du sel de sodium de sacubitril prêt à être comprimé;
- préparer éventuellement un mélange de couches de séparation ne contenant aucun principe actif;
- comprimer ledit mélange contenant d'hydrate de sel disodique de valsartan ou du valsartan sous forme d'acide libre, le mélange de sel de sodium de sacubitril et le mélange de séparation éventuel ne contenant aucun principe actif en un comprimé multicouche.
